# EUROPEAN PATENT SPECIFICATION

(11) **EP 3 976 162 B1**
(45) Date of publication and mention of the grant of the patent: **30.10.2024**
(21) Application number: 20721719.1
(22) Date of filing: 21.04.2020
(51) Int. Cl.: A61M 39/10, A61M 39/12

(54) **DIALYSER CONNECTOR**
DIALYSATOR-KONNEKTOR
CONNECTEUR DE DIALYSEUR

(30) Priority: 31.05.2019 GB 201907762
(43) Date of publication of application: 06.04.2022
(73) Proprietor: Quanta Dialysis Technologies Limited, Warwick CV34 5AH (GB)
(72) Inventor: FINCHAM, Sam, Alcester Warwickshire B49 6EU (GB)
(74) Representative: Range, Christopher William
(86) International application number: PCT/GB2020/050988
(87) International publication number: WO 2020/240150

(56) References cited:
- EP-A1- 0 183 396
- EP-A1- 3 006 072
- US-A- 4 123 091
- US-A1- 2013 060 268
- US-A1- 2015 051 536
- US-A1- 2019 022 368
- US-B2- 7 029 036

## Description

### TECHNICAL FIELD

The present disclosure relates to a dialyser connector. Particularly, but not exclusively, the disclosure relates to a dialyser connector comprising a main body and a sliding clip. Aspects of the invention relate to a dialyser connector, to a dialysate mixing and pumping cartridge comprising a dialyser connector, to a method of assembling a dialyser connector and to a method of connecting a dialyser connector to a dialyser filter connector port.

### BACKGROUND

A dialysis machine filters a patient's blood of toxins by pumping the blood through a disposable dialyser filter on one side of a semi-permeable membrane and pumping clean dialysate fluid through the disposable dialyser filter on the other side of the semi-permeable membrane. This allows the toxins to move across the semi-permeable membrane into the dialysate fluid and be removed from the blood. The blood and dialysate are pumped to and from the dialyser filter along fluid lines.

To connect the fluid lines to the connector ports of the dialyser filter, two types of connectors are used - one is a screw thread and the other is a push fit. The geometries of both of these types of connector ports are detailed in the standard ISO 8637-1.

Typically, dialysis machines use reusable versions of the push fit connectors while the screw fit connectors are part of the disposable blood tube sets. As the push fit connectors form part of the dialysate circuit, they require sterilization using hot water after each treatment and further chemical treatment on a regularly scheduled basis. As the push fit connectors are designed to be used many hundreds or thousands of times, these connectors are also designed to be robust, and are therefore expensive.

It is known to use a disposable cartridge to produce dialysate fluid and pump this through the dialyser filter. As a result, a disposable push fit connector is also required to fluidically connect the cartridge to the dialyser filter. Disposable push fit connectors are already available and are typically moulded as a single component from a flexible material which conforms around one of the connection ports on a dialyser filter. These connectors have the disadvantage of being very difficult to attach and remove and also may be prone to being pushed off by high pressures, especially after use over time. This invention relates to a new design of push fit dialyser connector which is inexpensive enough to be disposable but strong enough to be more reliable and convenient for a user. US2019/022368 discloses medical devices including vascular access kits and related methods. EP3006072 discloses a system (10; 710) for providing vascular access in a patient's body.

### SUMMARY OF THE INVENTION

The invention is defined by the subject-matter of the independent claims. Further embodiments are defined in the dependent claims. Aspects and embodiments of the invention provide a dialyser connector, a dialysate mixing and pumping cartridge comprising a dialyser connector, a method of assembling a dialyser connector and a method of connecting a dialyser connector to a dialyser filter connector port as claimed in the appended claims.

According to an aspect of the invention, there is provided a dialyser connector comprising a main body and a sliding clip, the main body has a tube end and a dialyser end, the main body defines a cavity, at least one aperture and a pair of ramps, and the sliding clip has a tube end and a dialyser end, the sliding clip defines a spine connecting the tube end and a dialyser end, the sliding clip has a pair of flexible wings extending in an arc from the spine and a pair of arms extending in an arc from the spine, the pair of arms have at least one projection extending radially inward, wherein the dialyser connector has a locked condition in which the at least one projection extends into the cavity of the main body and an unlocked conditions in which the at least one projection is withdrawn from the cavity of the main body, and wherein the at least one projection is biased to the locked condition by resilient abutment of pair of flexible wings on the pair of ramps.

The at least one projection may comprise a pair of projections and the at least one aperture may comprise a pair of apertures.

At least one arm of the pair of arms may have a hook configured to abut a shoulder of the main body to prevent detachment of the sliding clip from the main body when assembled.

The central portion may be bounded toward the tube end by a circumferential flange, preferably the central portion may be partially bounded toward the dialyser end by two arcuate flanges.

The spine may have a depression

The main body may have a projection, preferably wherein the spine has a notch.

The projection may be aligned with the vertical plane and extends from an upper surface of the main body.

The dialyser connector may further comprise a seal, preferably wherein the seal comprises an O-ring seal. Alternatively, the seal may comprise an overmould on the main body.

The dialyser connector may be symmetrical about a centred vertical plane.

The dialyser connector may be single use. The dialyser connector may be plastic.

One of the pair of arms may have a hinged latch. The hinged latch may be connected to the arm by a living hinge. The hinged latch may terminate in a wedge shape and the other of the pair of arms may have a corresponding ledge.

According to another aspect of the invention, there is provided a dialysate mixing and pumping cartridge comprising a dialyser connector as hereinbefore defined.

According to yet another aspect of the invention, there is provided a method of assembling a dialyser connector as hereinbefore defined, the method comprising the steps of: inserting an O-ring seal axially into the main body 30 from the dialyser end; mounting the sliding clip over the main body; slotting the pair of arms over the central portion with the at least one projection dropping into the at least one aperture with a snap fit connection, wherein the arm comprises a hook which abuts a shoulder of the main body to prevent detachment of the sliding clip from the main body when assembled.

According to a further aspect of the invention, there is provided a method of connecting a dialyser connector to a dialyser filter connector port, the method comprising the steps of providing a dialyser connector as hereinbefore defined, depressing the spine of the sliding clip relative to the main body so as to withdraw the at least one projection from the recess of a dialyser filter connector port, connecting the dialyser connector to the dialyser filter connector port, releasing the spine of the sliding clip relative to the main body so that the dialyser connector automatically assumes a locked condition in which the at least one projection engages a recess of a dialyser filter connector port under the biasing action of the pair of flexible wings on the pair of ramps.

The method may further comprise the step of disconnecting the dialyser connector from the dialyser filter connector port by depressing the spine of the sliding clip relative to the main body so as to withdraw the at least one projection from the recess of a dialyser filter connector port, and withdrawing the dialyser connector from the dialyser filter connector port.

### BRIEF DESCRIPTION OF THE DRAWINGS

One or more embodiments of the invention will now be described, by way of example only, with reference to the accompanying drawings, in which:

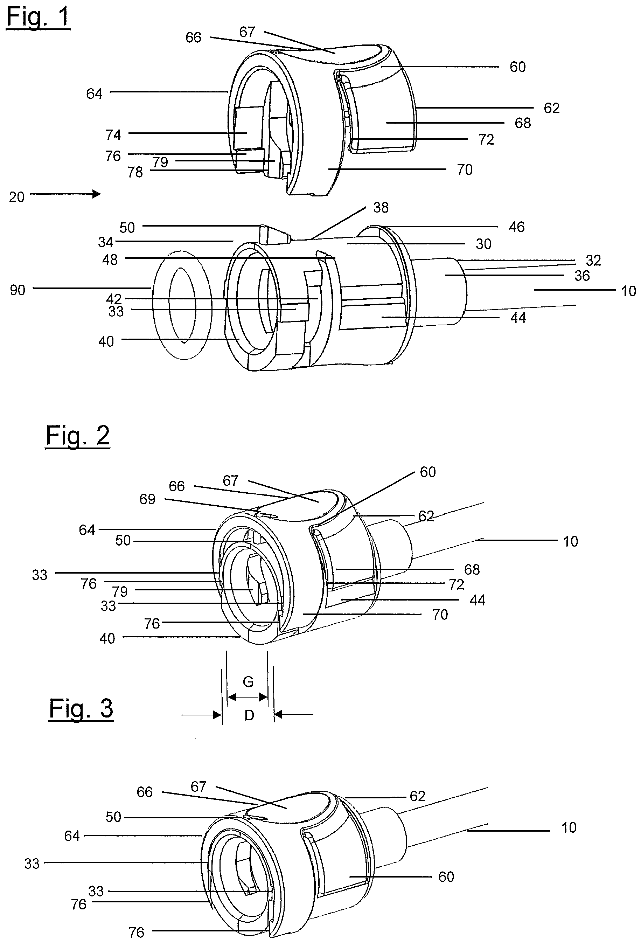

Figure 1 is an exploded schematic view of a dialyser connector according to an aspect of the invention;
Figure 2 is a schematic view of the dialyser connector of Figure 1 in a locked condition;
Figure 3 is a schematic view of the dialyser connector of Figure 1 in an unlocked condition;
Figure 4 is a cross-sectional view of the dialyser connector of Figure 1 in an unlocked condition;
Figure 5 is a partial section view of the dialyser connector of Figure 1 in a locked condition; and
Figure 6 is a schematic representation of the dialyser connector of Figure 1 in use;
Figure 7 is an exploded schematic view of a dialyser connector according to a further aspect of the invention;
Figure 8 is a schematic view of the dialyser connector of Figure 7 in an unlocked condition;
Figure 9 is a schematic view of the dialyser connector of Figure 7 in a locked condition; and
Figure 10 is a schematic view of the dialyser connector of Figure 7 in a locked condition.

### DETAILED DESCRIPTION

The dialyser connector 20 comprises a main body 30, a sliding clip 60 and an O-ring seal 90.

The dialyser connector 20 has a tube end 22 configured for connection to flexible dialysate tubing 10 and a dialyser end 24, configured for connection to a dialyser filter connector port 110, as will be described in more detail below. Looking end-on, in the orientation shown in all figures, the dialyser connector 20 is symmetrical about a vertical plane passing between the tube end 22 and the dialyser end 24.

The main body 30 is a single piece. The main body 30 defines a cylindrical cavity. The main body 30 has a tube end 32 and a dialyser end 34. Looking end-on, the main body 30 is symmetrical about a vertical plane passing between the tube end 32 and the dialyser end 34.

The tube end 32 and the dialyser end 34 are separated by a central portion 38. The tube end 32 terminates in a tube connector 36. The dialyser end 34 includes a shoulder 33 on each side. The dialyser end 34 terminates in an annulus 40. The annulus 40 is separated from the central portion 38 by two apertures 42. The two apertures 42 being provided on either side of the vertical plane.

The central portion includes a ramp 44 on either side, increasing a wall thickness of the central portion 38. The central portion 38 is bounded toward the tube end 32 by a circumferential flange 46. The central portion 38 is partially bounded toward the dialyser end 34 by two arcuate flanges 48.

A projection 50 extends from an upper surface of annulus 40. The projection is aligned with the vertical plane.

The sliding clip 60 is a single piece. The sliding clip 60 has an arcuate form, having a tube end 62 and a dialyser end 64. Looking end-on, the sliding clip 60 is symmetrical about a vertical plane passing between the tube end 62 and the dialyser end 64.

The tube end 62 and the dialyser end 64 are connected by a spine 66 running along the top of the sliding clip 60. The spine 66 has a depression 67. The spine has a notch 69, centrally aligned and toward the dialyser end 64. The tube end 62 comprises a pair of flexible wings 68 which extend in approximately 90 degree arcs from the spine 66, along either side of the sliding clip 60. The dialyser end 64 comprises a pair of arms 70 which extend in approximately 135 degree arcs from the spine 60, along either side of the sliding clip 60. The pair of flexible wings 68 are separated from the pair of arms 70 by respective cut-outs 72.

A dialyser end portion of an inside surface of each of the pair of arms 70 includes a cut-out 74 which defines a hook 76 adjacent the end of each arc. A tube end portion of the inside surface of each of the pair of arms 70 has a projection 78 adjacent the cut-outs 72. The projections 78 extend radially inward, along a portion of each arc. The projections 78 have a chamfer 79 facing the dialyser end 64.

### Assembly

The O-ring seal 90 is inserted axially into the main body 30 from the dialyser end 34, and retained in an annular recess 52 of an inside surface of the main body 30, adjacent the tube end 32.

The dialyser connector 20 is assembled with the sliding clip 60 mounted over the main body 30.

At the dialyser end 34, the pair of arms 70 are slotted over the central portion 38, with the projections 78 dropping into the respective apertures 42. This is a snap fit connection, the pair of arms 70 must flex outwardly when contacting the central portion 38, given that the gap G between the innermost points of the projections 78 is smaller than the distance D on the main body 30 between the apertures 42. Furthermore, each hook 76 of the pair of arms 70 abuts the shoulder 33 of the main body 30 to prevent detachment of sliding clip 60 from the main body 30. At the tube end 32, the pair of flexible wings 68 contact the ramp 44 on either side. The projections 78 impinge radially inwards through the apertures 42, and into the cavity of the main body, such that they are visible when viewing the dialyser connector 20 from the dialyser end 34.

The projection 50 extends through the notch 69 so that the projection is visible on the spine 66 of the sliding clip 60.

### Usage

Once assembled, the dialyser connector 20 may be manipulated between a locked condition in which the projections 78 engage a circumferential recess 112 of a dialyser filter connector port 110 and an unlocked conditions in which the projections 78 are withdrawn from the circumferential recess 112 of a dialyser filter connector port 110.

The contact of the ramps 44 on the pair of flexible wings 68 biases the dialyser connector 10 to the locked condition. Thus the projections 78 are biased to engage the circumferential recess 112 of the dialyser filter connector port 110.

Finger pressure upon the depression 67 of the spine 66 of the sliding clip 60 overcomes the biasing force as the pair of flexible wings 68 flex progressively up the inclined plane described by each ramp 44, allowing downward movement of the sliding clip 60 relative to the main body 30. The downward movement withdraws the projections 78 from impinging radially inwards through the apertures 42, and thus out of engagement with the circumferential recess 112 of a dialyser filter connector port 110. This allows removal of the dialyser connector 20 from the dialyser filter connector port 110.

Because of the resilient nature of the flexible wings 68, the dialyser connector 20 returns to the locked condition when the finger pressure is removed as the flexible wings 68 slide down the inclined plane described by each ramp 44, thus returning the flexible wings 68 to an un-flexed condition.

The projection 50 acts as an alignment feature to ensure that the dialyser connector 10 remains aligned vertically as it slides downwards. This also gives a visual indication to a user that the dialyser connector 20 has been fully depressed or that the dialyser connector 20 has fully sprung back to the locked condition.

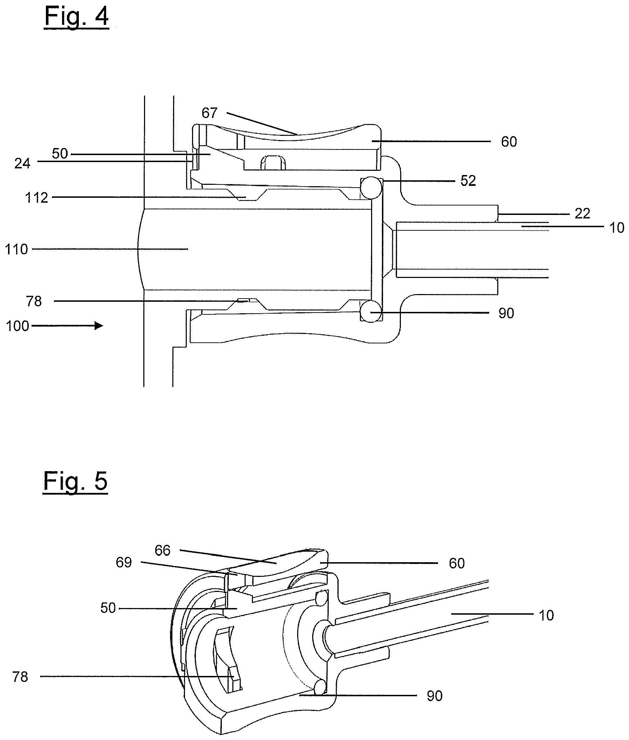

Referring to Figures 4 and 5, the O-ring seal 90 retained in an annular recess 52 of an inside surface of the main body 30, and abuts an outer end surface of the dialyser port 110. This provides a fluid tight seal between the dialyser connector 20 and the dialyser 100. The dialyser port 110 could be an inlet or and outlet of the dialyser 100.

The chamfer 79 on the projections 78 forces the sliding clip 60 downwards as it is pushed onto a dialyser port 110. This allows the connector 20 to be attached to a dialyser port 110 without pushing the sliding clip 60 down manually. The right angle on the rear of the projections 78 prevents the connector 20 from being removed without first pushing the sliding clip 60 down. Furthermore, the projection 50 maintains the sliding clip 60 straight as it slides down relative to the main body 30.

The dialyser connector 20 or the sliding clip 60 may be colour-coded to aid differentiation of the inlet and outlet connectors.

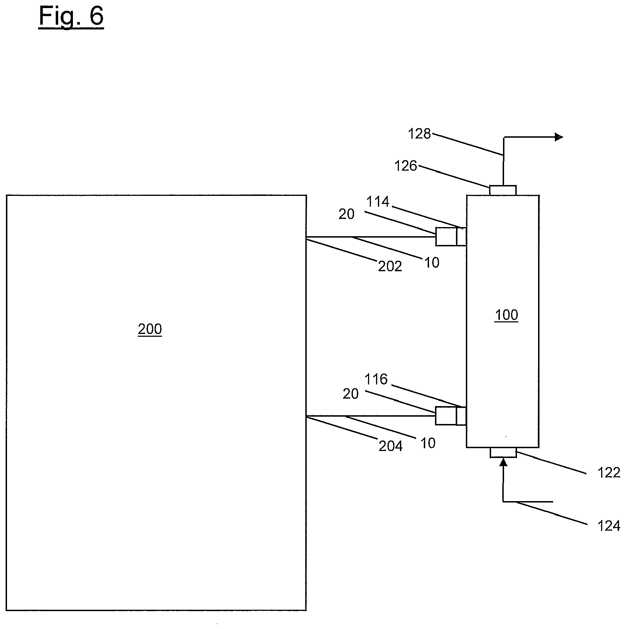

In an alternate embodiment, the seal is provided as an overmould on the main body 30. The overmould seal seals against the dialyser in the same manner as the O-ring seal 90. Figure 6 shows a schematic representation of the dialyser connector 20 in use with a disposable cartridge 200, for example the dialysate mixing and pumping cassette of WO 2010/146344 or the dialysate mixing and pumping cassette of WO 2013/110919.

The disposable cartridge 200 is responsible for pumping and mixing dialysate and has a clean dialysate outlet port 202 and a spent dialysis inlet port 204. These ports 202, 204 are fluidically connected to dialyser 100. Dialyser 100 has a blood inlet port 122 for receiving blood from arterial blood line 124 and blood outlet port 126 for sending blood to venous blood line 128. Dialyser 100 further has dialyser filter connector ports 110, in this case identified by dialysate inlet port 114 and dialysate outlet port 116. Two dialyser connectors 20 are used. One dialyser connector 20 fluidically connects clean dialysate outlet port 202 to the dialysate inlet port 114 using flexible dialysate tubing 10. Another dialyser connector 20 fluidically connects spent dialysate inlet port 204 to the dialysate outlet port 116 using flexible dialysate tubing 10. The flexible dialysate tubing 10 may be made from PVC. The flexible dialysate tubing 10 may be solvent bonded to the tube connector 36. In one embodiment, an outer surface of the PVC dialysate tubing 10 is coated with a mix of butanone and cycolohexanone and pushed into the tube connector 36. The solvent melts the two surfaces together, then evaporates to form a bond.

In an alternate embodiment, the tube end 22 of the dialyser connectors 20 terminate in a cartridge connector (not shown). The cartridge connector connects directly to the disposable cartridge 200, without the need for flexible dialysate tubing 10.

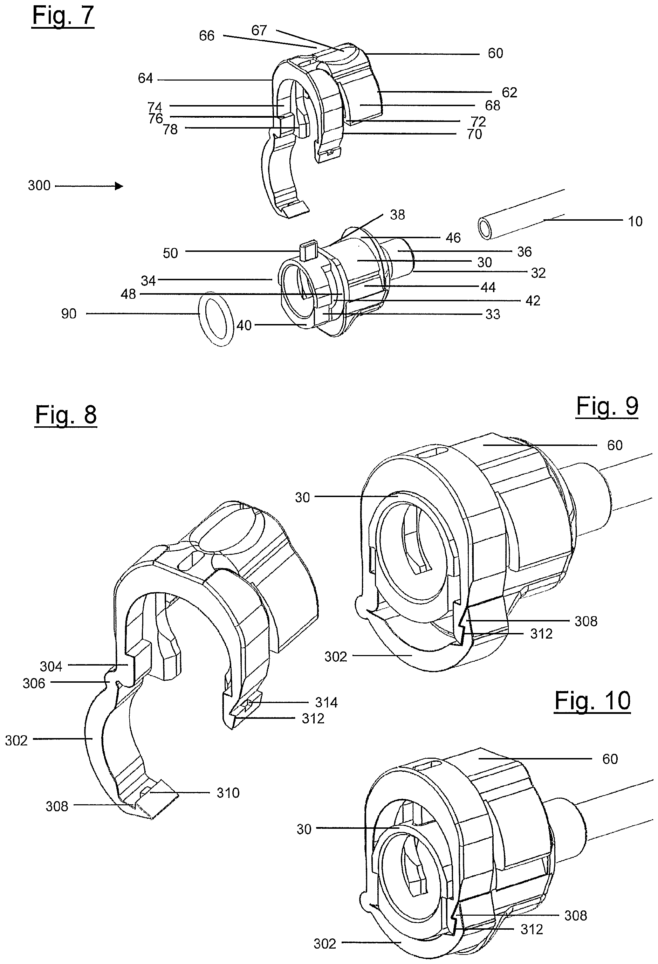

Figures 7 to 10 show an alternate embodiment of a dialyser connector generally designated 300. The dialyser connector 300 includes many of the features of dialyser connector 20 described above, only the main differences shall be described in detail.

Similar reference numbers are used to designate similar features as with respect to dialyser connector 20.

In the dialyser connector 300, one of the pair of arms 70 of the dialyser end 64 of the sliding clip 60 comprises a hinged latch 302. The hinged latch 302 extends from an end 304 of one the pair of arms 70 by means of a living hinge 306. The hinged latch 302 terminates in a wedge shape 308. The wedge shape 308 includes a projection 310. The other of the pair of arms 70 terminates in a corresponding ledge 312. The ledge 312 has a corresponding recess 314.

During use, the hinged latch 302 folds up and locks in place by means of the projection 310 engaging with recess 314, as the wedge shape 308 hooks over ledge 312.

The sliding clip 60 is still able to be moved relative to the main body 30, as per the dialyser connector 20 (see Figures 9 and 10).

The hinged latch 302 maintains the dialyser connector 300 firmly attached to the dialyser filter connector port 110. Furthermore, the hinged latch 302 prevents the pair of arms 70 from flexing outwards under excess pressure. The projection 310 and corresponding recess 314 provides an alignment feature which prevents the hinged latch 302 from axial movement relative to the ledge 312.

The hinged latch 302 may be released from engagement with the other of the pair of arms 70 by squeezing the pair of arms 70 together whilst stretching the hinged latch 302 to disengage the wedge shape 308 from ledge 312.

### List of Reference Numbers

| | | |
|---|---|---|
| - | flexible dialysate tubing | 10 |
| - | Dialyser connector | 20; 300 |
| - | tube end | 22 |
| - | dialyser end | 24 |
| - | main body | 30 |
| - | tube end | 32 |
| - | shoulder | 33 |
| - | dialyser end | 34 |
| - | tube connector | 36 |
| - | central portion | 38 |
| - | annulus | 40 |
| - | apertures | 42 |
| - | ramp | 44 |
| - | circumferential flange | 46 |
| - | arcuate flange | 48 |
| - | projection | 50 |
| - | annular recess | 52 |
| - | sliding clip | 60 |
| - | tube end | 62 |
| - | dialyser end | 64 |
| - | spline | 66 |
| - | depression | 67 |
| - | flexible wings | 68 |
| - | notch | 69 |
| - | pair of arms | 70 |
| - | cut-outs | 72 |
| - | cut-out | 74 |
| - | hook | 76 |
| - | projection | 78 |
| - | chamfer | 79 |
| - | O-ring seal | 90 |
| - | dialyser filter connector port | 110 |
| - | circumferential recess | 112 |
| - | dialysate inlet port | 114 |
| - | dialysate outlet port | 116 |
| - | blood inlet port | 122 |
| - | arterial blood line | 124 |
| - | blood outlet port | 126 |
| - | venous blood line | 128 |
| - | disposable cartridge | 200 |
| - | clean dialysate outlet port | 202 |
| - | spent dialysis inlet port | 204 |
| - | hinged latch | 302 |
| - | end | 304 |
| - | wedge shape | 308 |
| - | projection | 310 |
| - | ledge | 312 |
| - | recess | 314 |
| - | distance | D |
| - | gap | G |

## Claims

1. A dialyser connector (20,300) comprising:
a main body (30) and
a sliding clip (60),
the main body (30) has a tube end (22) and a dialyser end (34),
the main body (30) defines a cavity, at least one aperture (42) and a pair of ramps (44), and
the sliding clip (60) has a tube end (22) and a dialyser end (34),
the sliding clip (60) defines a spine connecting the tube end (22) and a dialyser end (34),
the sliding clip (60) has a pair of flexible wings (68) extending in an arc from the spine and a pair of arms (70) extending in an arc from the spine,
the pair of arms (70) have at least one projection (78) extending radially inward,
wherein the dialyser connector (20,300) has a locked condition in which the at least one projection extends into the cavity of the main body (30) and an unlocked conditions in which the at least one projection (78) is withdrawn from the cavity of the main body (30), and
wherein the at least one projection (78) is biased to the locked condition by resilient abutment of pair of flexible wings (68) on the pair of ramps (44).

2. A dialyser connector (20,300) according to claim 1, wherein the at least one projection (78) comprises a pair of projections (78) and wherein the at least one aperture (42) comprises a pair of apertures.

3. A dialyser connector (20,300) according to claim 1, wherein at least one arm of the pair of arms (70) has a hook (76) configured to abut a shoulder (33) of the main body (30) to prevent detachment of the sliding clip (60) from the main body (30) when assembled.

4. A dialyser connector (20,300) according to claim 1, wherein the central portion is bounded toward the tube end (22) by a circumferential flange (46), preferably wherein the central portion is partially bounded toward the dialyser end (34) by two arcuate flanges (48).

5. A dialyser connector (20,300) according to claim 1, wherein the spine has a depression (67).

6. A dialyser connector (20,300) according to claim 1, wherein the main body (30) has a projection (78), preferably wherein the spine has a notch (69).

7. A dialyser connector (20,300) according to claim 6, wherein the projection (78) is aligned with the vertical plane and extends from an upper surface of the main body.

8. A dialyser connector (20,300) according to claim 1, further comprising a seal (90), preferably wherein the seal (90) comprises an O-ring seal.

9. A dialyser connector (20,300) according to claim 8, wherein the seal (90) comprises an overmould on the main body (30).

10. A dialyser connector (20,300) according to any preceding claim, wherein the dialyser connector (20,300) is symmetrical about a centred vertical plane.

11. A dialyser connector according to any preceding claim, wherein the dialyser connector (20,300) is single use, preferably wherein the dialyser connector (20,300) is plastic.

12. A dialyser connector (20,300) according to any preceding claim, wherein one of the pair of arms (70) has a hinged latch (302).

13. A dialyser connector (20,300) according to claim 12, wherein the hinged latch (302) is connected to the arm (70) by a living hinge.

14. A dialyser connector (20,300) according to claim 12 or 13, wherein the hinged latch (302) terminates in a wedge shape and the other of the pair of arms (70) has a corresponding ledge (312).

15. A dialysate mixing and pumping cartridge comprising a dialyser connector (20,300) as defined in any of claims 1 to 14.

16. A method of assembling a dialyser connector (20,300) according to any of claims 1 to 14, the method comprising the steps of:
inserting an O-ring seal (90) axially into the main body (30) from the dialyser end (34);
mounting the sliding clip (60) over the main body (30);
slotting the pair of arms (70) over the central portion with the at least one projection (78) extending into the at least one aperture (42) with a snap fit connection, wherein the arm comprises a hook (76) which abuts a shoulder (33) of the main body (30) to prevent detachment of the sliding clip (60) from the main body (30) when assembled.

17. A method of connecting a dialyser connector (20,300) to a dialyser filter connector port, the method comprising the steps of providing a dialyser connector (20,300) as defined in any of claims 1 to 14,
depressing the spine of the sliding clip (60) relative to the main body (30) so as to withdraw the at least one projection (78) from the recess of a dialyser filter connector port (110),
connecting the dialyser connector to the dialyser filter connector port (110), releasing the spine of the sliding clip (60) relative to the main body (30) so that the dialyser connector (20,300) automatically assumes a locked condition in which the at least one projection (78) engages a recess of a dialyser filter connector port (110) under the biasing action of the pair of flexible wings (68) on the pair of ramps (44).

18. A method according to claim 17 further comprising the step of disconnecting the dialyser connector (20,300) from the dialyser filter connector port (110) by depressing the spine of the sliding clip (60) relative to the main body (30) so as to withdraw the at least one projection (78) from the recess of a dialyser filter connector port (110), and withdrawing the dialyser connector (20,300) from the dialyser filter connector port (110).

## Patentansprüche

1. Dialysator-Konnektor (20, 300), der aufweist:
einen Hauptkörper (30) und
einen Verschiebeclip (60),
wobei der Hauptkörper (30) ein Rohrende (22) und ein Dialysatorende (34) hat,
der Hauptkörper (30) einen Hohlraum, wenigstens eine Öffnung (42) und ein Paar von Schrägflächen (44) definiert, und
der Verschiebeclip (60) ein Rohrende (22) und ein Dialysatorende (34) hat,
wobei der Verschiebeclip (60) einen Rücken definiert, der das Rohrende (22) und ein Dialysatorende (34 ) verbindet,
der Verschiebeclip (60) ein Paar von flexiblen Flügeln (68), die in einem Winkel vom Rücken aus verlaufen, und ein Paar von Armen (70), die in einem Winkel vom Rücken aus verlaufen, hat,
das Paar der Arme (70) wenigstens einen Vorsprung (78) hat, der radial nach innen verläuft,
wobei der Dialysator-Konnektor (20, 300) einen gesperrten Zustand hat, in dem der wenigstens eine Vorsprung in den Hohlraum des Hauptkörpers (30) hineinsteht, und einen entsperrten Zustand, in dem der wenigstens eine Vorsprung (78) aus dem Hohlraum des Hauptkörpers (30) zurückgezogen ist, und
wobei der wenigstens eine Vorsprung (78) zum gesperrten Zustand durch elastisches Anliegen des Paars von flexiblen Flügeln (68) an dem Paar von Schrägflächen (44) vorgespannt ist.

2. Dialysator-Konnektor (20, 300) nach Anspruch 1,
wobei der wenigstens eine Vorsprung (78) ein Paar von Vorsprüngen (78) aufweist und wobei die wenigstens eine Öffnung (42) ein Paar von Öffnungen aufweist.

3. Dialysator-Konnektor (20, 300) nach Anspruch 1, wobei wenigstens ein Arm des Paar der Arme (70) einen Haken (76) hat, der ausgestaltet ist, an einer Schulter (33) des Hauptkörpers (30) anzuliegen, um ein Lösen des Verschiebeclips (60) aus dem Hauptkörper (30) im montierten Zustand zu verhindern.

4. Dialysator-Konnektor (20, 300) nach Anspruch 1,
wobei der Mittenbereich in Richtung des Rohrendes (22) durch einen Umfangsflansch (46) abgegrenzt ist, wobei vorzugsweise der Mittenbereich stellenweise in Richtung des Dialysatorendes (34) durch zwei angewinkelte Flansche (48) abgegrenzt ist.

5. Dialysator-Konnektor (20, 300) nach Anspruch 1,
wobei der Rücken eine Vertiefung (67) hat.

6. Dialysator-Konnektor (20, 300) nach Anspruch 1,
wobei der Hauptkörper (30) einen Vorsprung (78) hat, wobei der Rücken vorzugsweise eine Kerbe (69) hat.

7. Dialysator-Konnektor (20, 300) nach Anspruch 6,
wobei der Vorsprung (78) mit der vertikalen Ebene ausgerichtet ist und von einer Oberseite des Hauptkörpers aus verläuft.

8. Dialysator-Konnektor (20, 300) nach Anspruch 1,
der weiterhin eine Dichtung (90) aufweist, wobei die Dichtung (90) vorzugsweise eine O-Ring-Dichtung aufweist.

9. Dialysator-Konnektor (20, 300) nach Anspruch 8,
wobei die Dichtung (90) eine Ummantelung am Hauptkörper (30) aufweist.

10. Dialysator-Konnektor (20, 300) nach einem vorhergehenden Anspruch, wobei der Dialysator-Konnektor (20, 300) um eine vertikale Mittelebene symmetrisch ist.

11. Dialysator-Konnektor (20, 300) nach einem vorhergehenden Anspruch,
wobei der Dialysator-Konnektor (20, 300) zur Einmalverwendung ist, wobei der Dialysator-Konnektor (20, 300) vorzugsweise aus Plastik ist.

12. Dialysator-Konnektor (20, 300) nach einem vorhergehenden Anspruch,
wobei einer des Paars der Arme (70) einen Scharnierverschluss (302) hat.

13. Dialysator-Konnektor (20, 300) nach Anspruch 12,
wobei der Scharnierverschluss (302) mit dem Arm (70) durch ein Filmscharnier verbunden ist.

14. Dialysator-Konnektor (20, 300) nach Anspruch 12 oder 13,
wobei der Scharnierverschluss (302) in einer Keilform endet und der andere des Paars der Arme (70) einen entsprechenden Absatz (312) hat.

15. Dialysat-Misch- und Pump-Kassette, die einen Dialysator-Konnektor (20, 300) nach einem der Ansprüche 1 bis 14 aufweist.

16. Montageverfahren eines Dialysator-Konnektors (20, 300) nach einem der Ansprüche 1 bis 14, wobei das Verfahren die Schritte aufweist:
Einsetzen einer O-Ring-Dichtung (90) axial in den Hauptkörper (30) aus dem Dialysatorende (34);
Befestigen des Verschiebeclips (60) über dem Hauptkörper (30);
Einstecken des Paars der Arme (70) über dem Mittenbereich mit dem wenigsten einen Vorsprung (78), der in die wenigstens eine Öffnung (42) verläuft, mit einer Schnappverbindung, wobei der Arm einen Haken (76) aufweist, der an einer Schulter (33) des Hauptkörpers (30) anliegt, um ein Lösen des Verschiebeclips (60) aus dem Hauptkörper (30) im montierten Zustand zu verhindern.

17. Verbindungsverfahren eines Dialysator-Konnektors (20, 300) an einer Dialysator-Filterkonnektor-Anschlussöffnung, wobei das Verfahren die Schritte aufweist des Vorsehens eines Dialysator-Konnektors (20, 300) wie nach einem der Ansprüche 1 bis 14 definiert,
Herunterdrücken des Rückens des Verschiebeclips (60) bezüglich des Hauptkörpers (30), um so den wenigstens einen Vorsprung (78) aus der Aussparung einer Dialysator-Filterkonnektor-Anschlussöffnung (110) zurückzuziehen, Verbinden des Dialysator-Konnektors mit der Dialysator-Filterkonnektor-Anschlussöffnung (110), Lösen des Rückens des Verschiebeclips (60) bezüglich des Hauptkörpers (30), sodass der Dialysator-Konnektor (20, 300) automatisch einen gesperrten Zustand annimmt, in dem der wenigstens eine Vorsprung (78) in eine Aussparung einer Dialysator-Filterkonnektor-Anschlussöffnung (110) unter der Vorspannaktion des Paars von flexiblen Flügeln (68) am Paar der Schrägflächen (44) eingreift.

18. Verfahren nach Anspruch 17, das weiterhin den Schritt des Trennens des Dialysator-Konnektors (20, 300) aus der Dialysator-Filterkonnektor-Anschlussöffnung (110) aufweist, indem der Rücken des Verschiebeclips (60) bezüglich des Hauptkörpers (30) heruntergedrückt wird, um so wenigstens einen Vorsprung (78) aus der Aussparung der Dialysator-Filterkonnektor-Anschlussöffnung (110) zurückzuziehen und den Dialysator-Konnektor (20, 300) aus der Dialysator-Filterkonnektor-Anschlussöffnung (110) zurückzuziehen.

## Revendications

1. Connecteur de dialyseur (20, 300) comprenant :
un corps principal (30), et
une pince coulissante (60),
le corps principal (30) a une extrémité de tube (22) et une extrémité de dialyseur (34),
le corps principal (30) définit une cavité, au moins une ouverture (42) et une paire de rampes (44), et
la pince coulissante (60) a une extrémité de tube (22) et une extrémité de dialyseur (34),
la pince coulissante (60) définit une épine raccordant l'extrémité de tube (22) et une extrémité de dialyseur (34),
la pince coulissante (60) a une paire d'ailes flexibles (68) s'étendant sur un arc à partir de l'épine et une paire de bras (70) s'étendant sur un arc à partir de l'épine,
la paire de bras (70) a au moins une saillie (78) s'étendant radialement vers l'intérieur,
dans lequel le connecteur de dialyseur (20, 300) a une condition verrouillée dans laquelle la au moins une saillie s'étend dans la cavité du corps principal (30) et une condition déverrouillée dans laquelle la au moins une saillie (78) est retirée de la cavité du corps principal (30), et
dans lequel la au moins une saillie (78) est sollicitée dans la condition verrouillée par la butée résiliente de la paire d'ailes flexibles (68) sur la paire de rampes (44).

2. Connecteur de dialyseur (20, 300) selon la revendication 1, dans lequel la au moins une saillie (78) comprend une paire de saillies (78) et dans lequel la au moins une ouverture (42) comprend une paire d'ouvertures.

3. Connecteur de dialyseur (20, 300) selon la revendication 1, dans lequel au moins un bras de la paire de bras (70) a un crochet (76) configuré pour venir en butée contre un épaulement (33) du corps principal (30) pour empêcher le détachement de la pince coulissante (60) du corps principal (30) lors de l'assemblage.

4. Connecteur de dialyseur (20, 300) selon la revendication 1, dans lequel la partie centrale est délimitée vers l'extrémité de tube (22) par une bride circonférentielle (46), de préférence dans lequel la partie centrale est partiellement délimitée vers l'extrémité de dialyseur (34) par deux brides arquées (48).

5. Connecteur de dialyseur (20, 300) selon la revendication 1, dans lequel l'épine a une dépression (67).

6. Connecteur de dialyseur (20, 300) selon la revendication 1, dans lequel le corps principal (30) a une saillie (78), de préférence dans lequel l'épine a une encoche (69).

7. Connecteur de dialyseur (20, 300) selon la revendication 6, dans lequel la saillie (78) est alignée avec le plan vertical et s'étend à partir d'une surface supérieure du corps principal.

8. Connecteur de dialyseur (20, 300) selon la revendication 1, comprenant en outre un joint d'étanchéité (90), de préférence dans lequel le joint d'étanchéité (90) comprend un joint torique.

9. Connecteur de dialyseur (20, 300) selon la revendication 8, dans lequel le joint d'étanchéité (90) comprend un surmoulage sur le corps principal (30).

10. Connecteur de dialyseur (20, 300) selon l'une quelconque des revendications précédentes, dans lequel le connecteur de dialyseur (20, 300) est symétrique autour d'un plan vertical centré.

11. Connecteur de dialyseur selon l'une quelconque des revendications précédentes, dans lequel le connecteur de dialyseur (20, 300) est à usage unique, de préférence dans lequel le connecteur de dialyseur (20, 300) est en plastique.

12. Connecteur de dialyseur (20, 300) selon l'une quelconque des revendications précédentes, dans lequel l'un de la paire de bras (70) a un verrou articulé (302).

13. Connecteur de dialyseur (20, 300) selon la revendication 12, dans lequel le verrou articulé (302) est raccordé au bras (70) par une charnière active.

14. Connecteur de dialyseur (20, 300) selon la revendication 12 ou 13, dans lequel le verrou articulé (302) se termine par une forme de cale et l'autre bras de la paire de bras (70) a un rebord (312) correspondant.

15. Cartouche de mélange et de pompage de dialysat comprenant un connecteur de dialyseur (20, 300) selon l'une quelconque des revendications 1 à 14.

16. Procédé pour assembler un connecteur de dialyseur (20, 300) selon l'une quelconque des revendications 1 à 14, le procédé comprenant les étapes comprenant les faits de :
insérer un joint torique (90) de manière axiale dans le corps principal (30) à partir de l'extrémité de dialyseur (34) ;
monter la pince coulissante (60) sur le corps principal (30) ;
rainurer la paire de bras (70) sur la partie centrale avec la au moins une saillie (78) qui s'étend dans la au moins une ouverture (42) avec un raccordement par encliquetage, dans lequel le bras comprend un crochet (76) qui vient en butée contre un épaulement (33) du corps principal (30) pour empêcher le détachement de la pince coulissante (60) du corps principal (30) lors de l'assemblage.

17. Procédé pour raccorder un connecteur de dialyseur (20, 300) à un orifice de connecteur de filtre de dialyseur, le procédé comprenant les étapes comprenant les faits de prévoir un connecteur de dialyseur (20, 300) selon l'une quelconque des revendications 1 à 14,
enfoncer l'épine de la pince coulissante (60) par rapport au corps principal (30) afin de retirer la au moins une saillie (78) de l'évidement d'un orifice de connecteur de filtre de dialyseur (110),
raccorder le connecteur de dialyseur à l'orifice de connecteur de filtre de dialyseur (110), libérer l'épine de la pince coulissante (60) par rapport au corps principal (30) de sorte que le connecteur de dialyseur (20, 300) adopte automatiquement une condition verrouillée dans laquelle la au moins une saillie (78) met en prise un évidement d'un orifice de connecteur de filtre de dialyseur (110) sous l'action de sollicitation de la paire d'ailes flexibles (68) sur la paire de rampes (44).

18. Procédé selon la revendication 17, comprenant en outre l'étape comprenant le fait de déconnecter le connecteur de dialyseur (20, 300) de l'orifice de connecteur de filtre de dialyseur (110) en enfonçant l'épine de la pince coulissante (60) par rapport au corps principal (30) afin de retirer la au moins une saillie (78) de l'évidement d'un orifice de connecteur de filtre de dialyseur (110) et retirer le connecteur de dialyseur (20, 300) de l'orifice de connecteur de filtre de dialyseur (110).
